# FASCICULE DE BREVET EUROPEEN

(11) **EP 1 368 196 B1**
(45) Date de publication et mention de la délivrance du brevet: **04.06.2008**
(21) Numéro de dépôt: 02700351.6
(22) Date de dépôt: 22.01.2002
(51) Int. Cl.: B32B 37/00, A61F 13/15

(54) **PROCEDE DE PRODUCTION D'UNE STRUCTURE COMPOSITE STRATIFIEE A TOUCHER DOUX COMPRENANT AU MOINS DEUX COUCHES ET PRODUIT COMPOSITE STRATIFIE**
HERSTELLUNGSVERFAHREN UND PRODUKT, SCHICHTLAMINATE MIT WEICHER BERÜHRUNGSEIGENSCHAFT
METHOD FOR PRODUCING A LAMINATED COMPOSITE SOFT-FEEL STRUCTURE COMPRISING AT LEAST TWO LAYERS AND LAMINATE COMPOSITE PRODUCT

(30) Priorité: 22.01.2001 FR 0100831
(43) Date de publication de la demande: 10.12.2003
(73) Titulaire: Trioplanex France S.A., 80610 Saint Ouen (FR)
(72) Inventeur: GUSTAFSON, Bo, S-22655 Lund (SE)
(74) Mandataire: Gaucherand, Michel
(86) Numéro de dépôt international: PCT/FR2002/000242
(87) Numéro de publication internationale: WO 2002/057078

(56) Documents cités:
- EP-A- 0 568 812
- DE-A- 19 944 225
- GB-A- 1 394 824
- US-A- 5 421 921

## Description

### Domaine de l'invention

L'invention concerne un procédé continu de production d'une structure composite stratifiée laminée comprenant au moins deux couches dont l'une est un film thermoplastique imperméable aux liquides et l'autre est formée d'un substrat fibreux du type tissu non tissé, selon la préambule de la revendication 1 en plus, l'invention concerne une structure composite stratifiée selon la préambule de la revendication 42.

L'invention concerne plus particulièrement un procédé de production d'une structure composite stratifiée imperméable aux liquides et douce au toucher, comprenant au moins deux couches dont l'une est formée d'un film thermoplastique microporeux, imperméable aux liquides et l'autre est formée d'un tissu non tissé, cette structure composite stratifiée étant destinée à être utilisée dans la production d'articles d'hygiène jetables, tels que couches pour enfants, pour adultes incontinents ou articles d'hygiène féminine, et d'articles divers utilisés dans le domaine médical.

L'invention concerne plus spécifiquement un procédé de production d'une structure composite stratifiée flexible, douce au toucher, imperméable aux liquides et perméable à la vapeur d'eau et aux gaz, comprenant au moins deux couches dont l'une est un film thermoplastique imperméable aux liquide et perméable à la vapeur d'eau et aux gaz et l'autre est un substrat fibreux du type tissu non tissé, ces couches étant associées l'une à l'autre par leur lamination en présence d'un liant fibreux.

L'invention concerne très précisément un procédé de production d'une structure composite stratifiée flexible, douce au toucher, imperméable aux liquides mais perméable à la vapeur d'eau et aux gaz, comprenant au moins deux couches dont l'une est un film thermoplastique imperméable aux liquide et l'autre est un tissu non tissé, ces couches étant liées par l'intermédiaire de fibrilles d'adhésion produites in situ immédiatement avant que s'opère la lamination.

### Etat de l'art

Les films thermoplastiques mis en oeuvre en tant que couche externe dans la production d'articles d'hygiène jetables, tels que des couches absorbantes pour enfants, pour adultes incontinents ou articles d'hygiène féminine, doivent être conçus et réalisés de telle sorte que lesdits articles soient le plus confortables possible au porter et que leur contact avec la peau ne soit pas source d'irritations ou de blessures.

C'est pourquoi, de tels films thermoplastiques, qui sont naturellement imperméables aux liquides, sont préférentiellement rendus perméables aux gaz et, en particulier, à la vapeur d'eau, et doivent avoir souhaitablement un toucher aussi doux et agréable au porter que celui d'un textile destiné à être au contact direct avec la peau, tout en conservant des caractéristiques de bonne résistance et de durée.

Les couches externes des articles d'hygiène jetables pour enfants, adultes incontinents et articles d'hygiène féminine sont souvent réalisées au moyen de films polyoléfiniques, tels que en polyéthylène, imperméables aux liquides mais rendus perméables à la vapeur d'eau et au gaz par la création d'une microporosité plus ou moins importante, cette microporosité ayant la propriété de réduire l'accumulation simultanée de chaleur et d'humidité dans les zones de contact avec le corps humain.

Pour permettre d'améliorer leur toucher qui manque de douceur et rendre leur contact avec la peau équivalent à celui d'un textile particulièrement doux, ces couches externes des articles d'hygiène précités sont également réalisées au moyen d'une structure composite stratifiée, associant un film thermoplastique de plus en plus mince, respirable ou non respirable, mais imperméable aux liquides, à un composé fibreux, connu sous l'appellation de tissu non tissé pouvant avoir un toucher particulièrement doux et agréable au contact de la peau.

Diverses méthodes d'assemblage de plusieurs couches formant une structure composite stratifiée, c'est-à-dire comprenant un film thermoplastique et une structure fibreuse du type tissu non tissé, sont pratiquées qui consistent :
- soit en une extrusion-couchage ;
- ou une lamination avec adhésif spécifique
- ou une lamination à chaud.

Dans le cas d'une extrusion-couchage, un film fondu qui sert de liant est extrudé directement sur l'une des couches, en l'occurrence le tissu non tissé. L'autre couche formée du film à associer à la première couche est mis en contact avec le film fondu extrudé-couché au niveau du point de contact des deux rouleaux pinceurs alors que ledit film fondu est encore chaud, et est rendu adhésif par lamination des deux couches et du film fondu intermédiaire.

Dans le cas de la lamination avec adhésif spécifique, plus connue sous l'appellation hot-melt, l'une des couches à assembler est revêtue totalement
ou partiellement selon des zones à motifs organisés, par une colle distribuée à chaud par un moyen approprié. L'autre couche est alors mise en contact avec les zones de colle portées par la première couche, puis l'ensemble des deux couches est laminé pour créer leur adhésion.

Dans le cas de la lamination à chaud d'un film thermoplastique à associer par lamination au tissu non tissé, l'une ou l'autre de ces deux couches est formée en tout ou partie d'une composition telle que par chauffage, cette composition devient "collante" et adhère facilement à la surface de l'autre couche.

Les méthodes d'assemblage précédemment évoquées, appartenant à l'état de la technique, manifestent, les unes ou les autres, des inconvénients majeurs qu'il serait souhaitable de corriger, voire d'éliminer, dans la réalisation de structures composites stratifiées par assemblage de films thermoplastiques et de structures fibreuses telles que les non tissés.

Selon un premier inconvénient, s'il est possible, en effet, de réaliser, par l'une
ou l'autre de ces méthodes, une structure composite stratifiée composée d'un film thermoplastique et d'un tissu non tissé, le film thermoplastique qui constitue une barrière aux liquides, doit posséder une certaine épaisseur qui est sensiblement d'au moins 20 µm pour conserver cette caractéristique essentielle de barrière. En deçà de cette épaisseur de 20 microns, il est constaté que le film devient poreux et perd sa caractéristique de barrière aux liquides.

Selon un autre inconvénient, dès lors que ces méthodes d'assemblage nécessitent un film d'épaisseur au moins égale à 20 microns, deux caractéristiques essentielles, de plus en plus recherchées pour les structures composites stratifiées évoquées, qui sont d'une part la perméabilité aux gaz et à la vapeur d'eau et, d'autre part, un toucher textile, deviennent difficilement accessibles car ces caractéristiques nécessitent des films de très faible épaisseur, c'est-à-dire d'épaisseur souhaitablement inférieure à 20 microns.

Ainsi, la méthode d'assemblage par extrusion-couchage et lamination ne peut pas conduire à la production de structures composites stratifiées simultanément imperméables aux liquides, perméables à la vapeur d'eau et aux gaz et, enfin, ayant un toucher textile particulièrement doux, car, quelle que soit l'épaisseur du film thermoplastique à assembler, le polymère extrudé-couché, qui constitue le liant entre le tissu non tissé et le film à assembler, est fondu à une température élevée pour permettre l'abaissement de sa viscosité et permettre une enduction plus facile du substrat le recevant. Dès lors :
- lorsque le film à assembler avec le tissu non tissé a une épaisseur supérieure à 20 micromètres, la méthode d'assemblage par extrusion-couchage conduit à une structure composite stratifiée ayant un toucher à tendance rugueuse, non textile et présentant une perte importante de perméabilité aux gaz et à la vapeur d'eau ;
- lorsque le film à assembler avec le tissu non tissé a une épaisseur inférieure à 20 micromètres, ce film supporte mal la température élevée du polymère liant et les fortes pressions pratiquées en lamination pour obtenir la cohésion entre les couches lors de l'assemblage: la conséquence essentielle et dommageable, dans ce cas, est la détérioration du film assemblé, manifestée par l'apparition de petits trous ou déchirures qui affectent sérieusement l'imperméabilité aux liquides.

De même, quand est pratiquée la méthode d'assemblage par lamination à chaud d'un film d'une épaisseur inférieure à 20 micromètres et d'un tissu non tissé, ledit film est soumis à des température et pression élevées pour le ramollir et le faire pénétrer dans le substrat fibreux qui lui sont préjudiciables. En effet, que le film thermoplastique à assembler soit respirable ou non respirable, cette méthode d'assemblage entraîne une perte du toucher textile du tissu non tissé, avec apparition d'un toucher plus ou moins cartonneux pour la structure composite résultant de l'assemblage. De plus, si le film thermoplastique à assembler est respirable, cette méthode fait perdre simultanément tout ou partie de sa perméabilité aux gaz et à la vapeur d'eau. Enfin, pour que l'assemblage entre le film thermoplastique et le substrat fibreux puisse se faire selon cette méthode d'assemblage, les matières constitutives de ces deux couches doivent être compatibles entre elles ou rendues compatibles.

Quant à la méthode d'assemblage par lamination, en présence d'un adhésif spécifique (hot melt), d'un film thermoplastique et d'un substrat fibreux tel qu'un tissu non tissé, cette méthode pratique le dépôt de l'adhésif spécifique (hot melt), préalablement à la lamination :
- soit sur la totalité de la surface de l'une des couches à assembler. Ce dépôt peut être réalisé par l'intermédiaire d'une filière à fente sans contact avec l'une des couches à assembler ou d'un cylindre de dépôt ayant un état de surface approprié ;
- soit sur une partie seulement de la surface totale, par la création de zones ou points de collage organisés par l'intermédiaire d'un rouleau ayant un état de surface approprié telle que gravure, ou par la pulvérisation discontinue et organisée de l'adhésif spécifique au moyen de buses ;
- soit par pulvérisation aléatoire dudit adhésif spécifique sur le substrat, ou par extrusion de fils thermoplastiques au-dessus du substrat et par le dépôt de ces fils sur le substrat sous une forme spiralée.

Quand le dépôt de l'adhésif spécifique (hot melt) est réalisé sur la totalité de la surface de l'une des couches à assembler, alors que le film thermoplastique a une épaisseur inférieure à 20 microns, la structure composite stratifiée résultant de l'assemblage des touches par lamination, en présence dudit adhésif spécifique, révèle une perte de l'imperméabilité aux liquides, ainsi que l'apparition d'un toucher rugueux, désagréable et irritant dans les applications entraînant un contact avec le corps humain et, dans le cas où le film thermoplastique à assembler est microporeux, d'une diminution de la perméabilité aux gaz et à la vapeur d'eau.

Quand la dépôt de l'adhésif spécifique (hot melt) est réalisé selon des zones organisées ou des points de collage à géométrie déterminée, cette méthode nécessite la mise en oeuvre de l'adhésif spécifique ayant un état de surface approprié par un rouleau ou par des buses de pulvérisation, l'un et l'autre moyens exigeant des adhésifs spécifiques de composition particulièrement adaptés à chaque cas.

Quand le dépôt de l'adhésif spécifique (hot melt) est réalisé par pulvérisation aléatoire sur le substrat au moyen de buses ou par extrusion de fils au moyen d'une filière placée au-dessus de substrat, ces fils se déposant sous une forme de spirales sur le substrat. Ces deux méthodes nécessitent des aménagements technologiques complexes, , des filières d'extrusion particulières et des méthodes de fonctionnement spécifiques et compliqué, en particulier pour ces filières. Le brevet GB 1 394 824 décrit d'une manière générale des techniques pour former des sandwiches constitués de deux feuilles polymères externes et une couche en matériau thermofusible capable de former des fibres.

A travers les publications évoquées ci-après, certaines méthodes d'assemblage par lamination d'un film thermoplastique et d'un tissu non tissé au moyen d'un adhésif spécifique (hot melt) peuvent être illustrées.

Par exemple :
- le brevet US-5,000,112 décrit une méthode de dépôt d'un adhésif spécifique (hot melt), selon un faible grammage, sur la totalité de la surface d'un substrat à partir d'une filière à fente d'environ 80 centimètres de large, alimentée par une pluralité de buses. Une telle installation rend difficile un changement de largeur du substrat qui reçoit l'adhésif. En outre, cette installation produit, pour la largeur donnée, une structure laminée dont le toucher est relativement rugueux.
- le brevet EP0568812 décrit une méthode de dépôt d'un adhésif spécifique (hot melt), selon un faible grammage, sur la surface d'un substrat à partir d'une pluralité de buses, pilotées séparément en ce qui concerne leur alimentation. Il est clair que cette méthode est compliquée et particulièrement délicate de fonctionnement dans une production industrielle qui nécessite, de préférence, des installations les plus simples possible, robustes et reproductives.
- le brevet EP0774909 décrit une méthode de dépôt d'un adhésif spécifique (hot melt) par la formation de zones d'adhésion délimitées, ayant une configuration géométrique organisée. A l'échelle industrielle, ce type d'installation est complexe à mettre en oeuvre, et ne peut fonctionner facilement à des vitesses de défilement et d'assemblage élevées.
- Le brevet DE 19944225 décrit des méthodes de dépôt d'un adhésif « hot melt » du type polyalphaoléfine amorphe (APAO) dont l'une consiste en l'atomisation du « hot melt », c'est-à-dire la pulvérisation du « hot melt » fondu sur le substrat à traiter au moyen de buses et dont une autre consiste en la production de fils à partir d'une technologie appropriée placée au-dessus du substrat à traiter, les fils formés se déposant sur ce substrat sous forme de spirales. En pratique, le traitement préconisé de préparation de la surface d'un substrat par dépôt d'un « hot melt » sous forme d'une pulvérisation ou de filaments spiralés en vue de son assemblage par laminage avec un autre substrat, conduit à un laminé dont les couches sont de collage faible, insuffisant en particulier lorsque ce laminé est mis en oeuvre dans des articles soumis à des efforts mécaniques, comme des articles d'hygiène.
- Le brevet US 5,421,921 décrit un dispositif et un procédé destinés à fabriquer un tissu fibreux adhésif sur un substrat, en vue de l'assembler avec un autre substrat par laminage, par exemple.

Le dispositif est une filière à fente comportant une buse d'injection dans un plan perpendiculaire à la surface du substrat à traiter et au sens de marche dudit substrat et deux buses obliques d'air comprimé chaud débouchant sur l'extrémité de la buse du « hot melt », placées de part et d'autre de ladite buse du « hot melt ».

Quant au procédé de production d'un tissu fibreux par dépôt de fibres adhésives sur un substrat, ce procédé comprend les étapes d'alimentation en « hot melt » d'une fente de filière située au-dessus du substrat, de séparation du « hot melt » dans la fente de filière en une pluralité de courants de « hot melt », et de réunion de ces courants, dès la sortie de la fente pour former un « rideau » de « hot melt » fondu, et enfin d'alimentation en air comprimé chaud, des buses d'air placées de chaque côté dudit rideau de « hot melt » pour produire les fibres adhésives sans qu'il y ait contact de la filière avec ie substrat devant recevoir les fibres adhésives. Outre qu'il est question d'une filière tout à fait particulière, il est également question de l'usage d'air comprimé pour la formation des fibres adhésives, sans lequel un rideau de « hot melt » fondu recouvrirait totalement le substrat à traiter sous l'aspect d'un film et sans lequel la formation de fibres adhésives est impossible. Ainsi, la complexité de la filière et la nécessité de mettre en oeuvre de l'air comprimé rendent le dispositif et le procédé peu à même de répondre à un besoin industriel qui recherche, plus volontiers, des moyens simples, mis en oeuvre selon des méthodes préférentiellement innovantes et également simples.

### Objet de l'invention

Dès lors, l'invention poursuit, à travers des objectifs précis, la création d'une structure composite stratifiée exempte des inconvénients précités et disposant même de caractéristiques très améliorées par rapport à celles révélées par l'état de la technique, ainsi que la création d'un procédé de production de cette structure composite stratifiée.

Un premier objet ciblé par l'invention est la création d'une structure composite stratifiée associant au moins un film thermoplastique imperméable aux liquides et perméable ou non aux gaz et à la vapeur d'eau et un substrat fibreux du type tissu non tissé.

Un autre objet ciblé par l'invention est la création d'une structure composite stratifiée, au toucher doux, similaire au toucher d'un textile, d'une bonne résistance mécanique et d'une grande souplesse.

Un autre objet ciblé par l'invention est la création d'une structure composite stratifiée composée d'au moins deux couches dont l'une est formé d'un film thermoplastique, respirable ou non, et l'autre est formée d'un substrat fibreux non tissé, pouvant être utilisé en particulier dans les articles d'hygiène jetables et obtenue à grande vitesse, selon un procédé continu de lamination.

Un autre objet ciblé par l'invention est la création d'une structure composite stratifiée, obtenue par lamination dont le moyen d'assemblage des diverses couches est fait par un adhésif spécifique de type hot melt ne recouvrant qu'une faible partie de la surface totale de la couche de réception.

Un autre objet ciblé par l'invention est la création d'une structure composite stratifiée, composée d'au moins deux couches dont l'une des couches est le siège de la formation in situ de fibrilles adhésives thermoplastiques de type « hot melt », immédiatement avant laminage avec une autre couche.

Un autre objet ciblé par l'invention est la création d'un procédé continu de formation in situ de fibrilles adhésives thermoplastiques, de type « hot melt » sur l'une des deux couches à assembler par laminage immédiatement avant laminage de deux couches.

Un autre objet ciblé par l'invention est la création d'un procédé continu de formation in situ de fibrilles adhésives thermoplastique de type « hot melt » sur une couche en mouvement continu par contact physique direct et contrôlé avec ladite couche du moyen de formation in situ par extrusion, immédiatement avant assemblage par laminage avec une autre couche.

### Sommaire de l'invention

Conformément aux divers objets précédemment évoqués, le procédé selon l'invention est non seulement susceptible d'éliminer les inconvénients évoqués lors de l'examen de l'état de la technique, mais également d'apporter de nombreux avantages inexistant dans les procédés décrits dans l'état de la technique.

Ainsi, l'invention concerne un procédé de production d'une structure composite stratifiée laminée comprenant au moins deux couches dont l'une est formée d'un film thermoplastique imperméable aux liquides et l'autre est formée d'un substrat fibreux du type tissu non tissé, les couches étant rendues adhérentes entre elles par leur laminage en présence d'un liant qui se caractérise en ce que le moyen liant est formé par des fibrilles d'adhésion produites in situ à partir d'au moins un polymère thermoplastique sur l'une des couches immédiatement avant que s'effectue la lamination des au moins deux couches, par un moyen de formation par extrusion en contact physique contrôlé avec la couche recevant les fibrilles.

L'invention concerne également une structure composite stratifiée comprenant au moins deux couches dont l'une est formée d'un film thermoplastique imperméable aux liquides et l'autre est formée d'un substrat fibreux du type tissu non tissé, les couches étant rendues adhérentes entre elles par laminage en présence d'un liant qui se caractérise en ce que ce liant est constitué par des fibrilles d'adhésion produites in situ sur l'une des couches par le contact physique et contrôlé, du moyen de formation par extrusion desdites fibrilles avec la couche recevant les fibrilles immédiatement avant le laminage des couches.
Des modes de réalisation particuliers de l'invention font l'objet des revendications dépendantes.

### Description détaillée de l'invention

Selon l'invention, les au moins deux couches de la structure composite stratifiée sont rendues adhérentes entre elles par une opération de laminage d'au moins un film thermoplastique imperméable aux liquides avec au moins un substrat fibreux formé d'un tissu non tissé en présence d'un liant d'adhésion constitué de fibrilles adhésives thermoplastiques produites in situ sur l'une des couches par le contact physique et contrôlé du moyen de formation par extrusion de fibrilles, avec cette couche, siège de la formation in situ desdites fibrilles immédiatement avant que s'effectue la lamination.

Ainsi, les au moins deux couches de la structure composite stratifiée selon l'invention sont rendues adhérentes entre elles par la production in situ de fibrilles adhésives thermoplastiques sur l'une des couches, qui est préférentiellement la couche fibreuse, suivie du laminage sous pression desdites couches entre lesquelles se situent lesdites fibrilles.

### Caractéristiques des fibrilles adhésives

Les fibrilles adhésives selon l'invention, produites in situ, sont préférentiellement formées à partir de polymères et/ou copolymères thermoplastiques appartenant au groupe constitué par les polymères et/ou copolymères amorphes ou non de styrène-isoprène-styrène, de styrène-butadiène-styrène, d'éthylène et propylène, d'éthylène et/ou de propylène avec au moins une alpha oléfine en C₄ à C₁₀, telle que éthylène-butène, propyléne-hexène, utilisés seuls ou en mélange.

Plus précisément, les fibrilles adhésives produites in situ selon l'invention sont souhaitablement formées d'au moins 20% en poids, et préférentiellement de 30% à 100% en poids, de poly-α-oléfines amorphes appartenant au groupe constitué par les copolymères d'éthylène et propylène amorphes, par les copolymères d'éthylène et d'anciennes en C₄ à C₁₀ amorphes et les copolymères de propylène et d'α-oléfines en C₄ à C₁₀ amorphes, utilisés seuls
ou en mélange.

Les fibrilles adhésives, produites in situ, peuvent également contenir, dans leur composition, des adjuvants tels que des adhésifs (tackiflants), des antioxydants, des plastifiants où autres.

La composition destinée à la formation des fibrilles adhésives produites in situ dispose souhaitab!ement d'une viscosité à t'état fondu comprise entre 2500 et 3000 mPa.s et préférentiellement comprise entre 2700 et 2900 mPa.s, pour une température de 175° C (mesurée avec un viscosimètre Brookfield RVT).

En deçà d'une viscosité de 2500 mPsa, ou au-delà d'une viscosité de 3000 mPa·s, mesurée à 175° C, pour une composition destinée à la formation de fibrilles adhésives in situ, il devient difficile de former les fibrilles adhésives, la composition fondue étant, dans le premier cas, trop liquide et, dans le deuxième cas, trop pâteuse pour permettre la formation, adéquate desdites fibrilles adhésives, des gouttes et/ou bandelettes pouvant se former, donnant à la structure composite stratifiée un effet cartonneux par zone et un risque de délaminage des couches par un manque de régularité dans leur adhésion.

Les fibrilles adhésives, produites in situ selon l'invention, ont une longueur souhaitablement d'au plus 20 mm et préférentiellement comprises entre 0,1 et 10 mm, et un rapport entre leur longueur et leur largeur après lamination comprise entre 1 et 100.

### Mode de formation des fibrilles adhésives

Selon l'invention, les fibrilles adhésives constituant le moyen d'adhésion des au moins deux couches formant, par laminage, la structure composite stratifiée :
- l'une des couches au moins étant un film thermoplastique imperméable aux liquides, perméable ou non aux gaz et à la vapeur d'eau ;
- et l'autre couche au moins étant un substrat fibreux tel qu'un tissu non tissé ;
sont produites in situ, préférentiellement sur le substrat fibreux, dans le sens de son défilement, immédiatement avant que s'effectue le laminage des couches.

Le moyen de formation desdites fibrilles adhésives est tel que ces fibrilles produites à l'état fondu sur l'une des couches, en particulier sur le substrat fibreux :
- ont une longueur distribuée de façon aléatoire à l'intérieur des limites précédemment évoquées, c'est-à-dire dont la longueur est souhaitablement d'au plus 20 mm ;
- et sont distribuées de façon aléatoire sur toute la surface de la couche les recevant, sans occuper la totalité de cette surface.

Le moyen de formation préférentiellement utilisé pour la production in situ des fibrilles adhésives immédiatement avant que s'effectue la lamination des diverses couches, est préférentiellement une filière à fente, alimentée par la composition ayant les caractéristiques précédemment énoncées, et destinée à la formation desdites fibrilles adhésives.

La formation in situ des fibrilles adhésives sur l'une des couches et préférentiellement sur la couche fibreuse, est obtenue par la combinaison :
- de l'alimentation du moyen de formation des fibrilles, qui est, par exemple, la filière à fente, avec la composition destinée à cette formation, ledit moyen de formation étant sous-alimenté en composition destinée à la formation des fibrilles adhésives in situ;
- de la vitesse linéaire de défilement de la couche recevant les fibrilles, qui peut être préférentiellement un substrat fibreux du type tissu non tissé ;
- du contact physique contrôlé du moyen de formation des fibrilles adhésives, c'est-à-dire la filière à fente par exemple, avec le substrat fibreux, siège de la formation desdites fibrilles,
de telle sorte que la quantité en poids de fibrilles formées in situ par unité de surface soit bien contrôlée.

La sous-alimentation du moyen de formation des fibrilles définit un débit de la composition destinée à la formation in situ des fibrilles adhésives, en relation avec la vitesse de défilement de la couche recevant lesdites fibrilles, de telle sorte que la quantité en poids de fibrilles adhésives produites in situ sur ladite couche en défilement, exprimée en gramme par mètre carré, recouvre au plus 20%, et préférentiellement de 5% à 10%, de la surface totale de la couche en défilement recevant lesdites fibrilles.

La quantité, exprimée en poids, de fibrilles formées in situ est d'au plus 6 g/m² et est préférentiellement comprise entre 1,5 g/m² et 2,5 g/m².

Dans le cas où la quantité de fibrilles adhésives formées in situ est inférieure à 1,5 g/m², l'adhésion des couches constitutives de la structure composite stratifiée peut être insuffisante et provoquer quelques effets accidentels de délaminage et, dans le cas où la quantité de fibrilles adhésives formées in situ est supérieure à 6 g/m², la structure composite stratifiée obtenue présente un toucher infiniment moins doux, qui n'est plus un toucher textile, une perte de perméabilité aux gaz et à la vapeur d'eau, quand le film thermoplastique est initialement microporeux, et un effet d'engorgement au niveau de la filière à fente.

Quant à la vitesse linéaire de défilement de la couche recevant les fibrilles, préférentiellement la couche fibreuse formée d'un tissu non tissé, cette vitesse est comprise entre 50 m/min et 500 m/min et est préférentiellement comprise entre 150 m/min et 300 m/min. Dans le cas où la vitesse linéaire de défilement est faible, par exempte inférieure à 50 m/min, la formation in situ des fibrilles adhésives, et leur distribution au hasard sur la surface de la couche les recevant, restent possible mais cette vitesse, trop lente, est dissuasive sous l'aspect économique d'un procédé industriel, le laminage des couches formant la structure composite stratifiée étant peu productif.

A l'opposé, c'est-à-dire quand la vitesse de défilement est supérieure à 500 m/min, la formation in situ des fibrilles adhésives, bien que également possible, n'est pas particulièrement recommandable car apparaissent des inconvénients dans la formation de fibrilles adhésives, ces inconvénients se manifestant dans le caractère moins aléatoire de leur longueur et de leur distribution sur la surface de la structure fibreuse qui est mal maîtrisée, ainsi que dans le comportement de la couche et, plus particulièrement, du substrat fibreux recevant les fibrilles adhésives qui est soumis à des tensions plus fortes.

Outre ces deux premières caractéristiques que sont la sous-alimentation de la filière à fente en composition utile pour la formation in situ des fibrilles adhésives et la vitesse linéaire de défilement de la couche recevant les fibrilles, la formation des fibrilles adhésives in situ est également dépendante, pour partie, d'un contact physique contrôlé entre le moyen de formation in situ des fibrilles adhésives, telle que la filière à fente, et la couche qui reçoit les fibrilles adhésives formées au moyen de la composition polymère à l'état fondu.

Le contact contrôlé entre le moyen de formation in situ des fibrilles adhésives et la couche en défilement linéaire qui reçoit les fibrilles adhésives peut être obtenu par une position spatiale spécifique du moyen de formation in situ des fibrilles. Cette position spatiale spécifique peut se définit par l'intermédiaire de deux plans concourants dont l'intersection se situe sur la couche recevant les fibrilles adhésives perpendiculairement au sens de défilement de ladite couche. Ces deux plans forment un dièdre d'angle α dont le premier plan, qui passe par le moyen de formation des fibrilles, est perpendiculaire à l'axe de symétrie de la couche recevant les fibrilles et le deuxième plan est simultanément perpendiculaire à l'axe de symétrie de la couche recevant les fibrilles et à la surface de défilement de cette même couche.

L'angle α positif ainsi défini par ces deux plans concourants formant dièdre peut prendre une valeur de l'intervalle entre 10° et 0° et préférentiellement entre 2° et 0° en amont et par rapport à ce deuxième plan.

Un angle α négatif, qui serait formé en aval du deuxième plan précédemment défini, ne conduirait plus à la seule formation de fibrilles in situ, mais également à la formation de gouttes, de bandes absolument préjudiciables à la qualité finale de la structure composite stratifiée résultant du laminage en présence des fibrilles adhésives produites in situ.

De plus, le contact contrôlé entre le moyen de formation in situ des fibrilles adhésives et la couche en défilement linéaire recevant lesdites fibrilles produites in situ peut comporter la création d'une indentation dans ladite couche en mouvement par une position spatiale particulière au moyen de formation in situ de fibrilles adhésives. Cette indentation peut résulter de la transformation d'un contact tangentiel dudit moyen de formation avec la couche recevant les fibrilles produites in situ en un contact déformant la surface plane de ladite couche en mouvement par l'abaissement et la prise d'appui dudit moyen de formation sur cette couche : la surface initialement plane de ladite couche recevant les fibrilles adhésives est remplacée par une surface descendante en amont de la prise d'appui du moyen de formation sur ladite couche, et par une surface ascendante en aval de ladite prise d'appui, le changement de pente se produisant au niveau du contact entre le moyen de formation in situ des fibrilles et la couche recevant lesdites fibrilles adhésives produites in situ.

La profondeur de l'indentation dans la couche recevant les fibrilles adhésives par l'abaissement et la prise d'appui du moyen de formation in situ des fibrilles sur ladite couche peut varier entre 0 et 5 mm et, préférentiellement, entre 2 et 3 mm.

D'une manière concomitante, la couche en défilement recevant les fibrilles adhésives, et qui est en contact contrôlé avec le moyen de formation in situ desdites fibrilles, est soumise à une tension contrôlée, généralement appliquée entre deux groupes de rouleaux d'entraînement, le premier groupe de rouleaux étant placé en amont du moyen de formation in situ des fibrilles, le deuxième groupe de rouleaux étant placé en aval dudit moyen. La tension contrôlée appliquée à ladite couche en défilement est comprise dans l'intervalle de 500 g à 2000 g et préférentiellement de 800 g à 1500 g.

La température à laquelle est soumise la composition polymère destinée à la formation in situ des fibrilles adhésives est comprise entre 120° C et 185° C, mais préférentiellement comprise entre 140° C et 165° C.

Selon l'invention, comme précédemment évoqué, la surface de la couche en défilement recouverte par les fibrilles adhésives produites in situ selon le procédé de l'invention représente au plus 20% de la surface totale de ladite couche. D'une manière plus particulière, la surface de ladite couche recouverte de fibrilles adhésives produites in situ selon le procédé représente préférentiellement de environ 5% à environ 10% de la surface totale de ladite couche en défilement.

Quand la surface de la couche recouverte par les fibrilles adhésives produites in situ selon le procédé devient supérieure à 20% de la surface totale, la structure composite stratifiée résultant du laminage tend à manifester un toucher plus cartonneux avec l'apparition d'une perte de souplesse et, quand le film est microporeux, d'une perte de perméabilité aux gaz et à la vapeur d'eau.

A contrario, quand la surface de la couche recouverte par les fibrilles adhésives produites in situ est inférieure à 2%, la cohésion entre les diverses couches formant la structure composite stratifiée diminue et peut donner une amorce de délaminage desdites couches constitutives.

Selon le procédé de l'invention, les fibrilles adhésives produites in situ, immédiatement avant le laminage des couches constitutives de la structure composite stratifiée, sont orientées longitudinalement sur la couche fibreuse, c'est-à-dire dans le sens de défilement de ladite couche.

### Film thermoplastique imperméable aux liquides

Le film thermoplastique intervenant dans la structure composite stratifiée selon l'invention est un film imperméable aux liquides selon l'utilisation envisagée, qui peut être ou non microporeux, c'est-à-dire perméable ou non aux gaz et à la vapeur d'eau.

Le film thermoplastique mis en oeuvre selon l'invention peut être un film monocouche ou multicouches dont les diverses couches peuvent être de même composition ou de composition différente répondant à des structures telles que, par exemple, A-A, A-B, B-C, A-B-C, A-B-A, C-B-C, ou autres.

Le film thermoplastique mis en oeuvre selon l'invention a une épaisseur souhaitablement comprise entre 10 µm et 30 µm, et préférentiellement comprise entre 13 µm et 20 µm.

Le film thermoplastique mis en oeuvre selon l'invention peut être formé à partir de polymères et/ou copolymères de compositions diverses et connues telles que, par exemple, des polyoléfines, des polyesters, des polyuréthanes, les alcools polyvinyliques, ou autres. Toutefois, les compositions les plus fréquemment utilisées pour la production de ces films thermoplastiques sont celles développées à partir des résines polyoléfiniques qui peuvent, par exemple, être issues du groupe constitué par les polyéthylènes de haute densité, les polyéthylènes de basse densité, lés copolymères d'éthylène et d'α-oléfines en C₄ à C₁₀, les polypropylènes, les copolymères de propylène et d'α-oléfines en C₄ à C₁₀, les copolymères d'éthylène-propylène.

Quand le film thermoplastique mis en oeuvre selon l'invention est perméable aux gaz et à la vapeur d'eau, la microporosité du film peut être obtenue par l'intermédiaire des moyens bien connus de l'état de la technique dont, en particulier, le moyen comportant l'extension d'un film formé à partir d'un mélange fondu d'au moins un polymère et/ou un copolymère thermoplastique et d'une charge minérale, suivie d'une action d'étirage monoaxiale ou biaxiale.

La perméabilité du film thermoplastique mis en oeuvre selon l'invention est souhaitablement comprise entre 500 g/m²/24 h et 5000 g/m²/24 h, et préférentiellement comprise entre 2000 g/m²/24 h et 5000 g/m²/24 h, cette perméabilité étant mesurée sous les conditions de température de 38° C et d'humidité relative de 90% (selon la méthode "Lyssy apparatus model 5000").

### Substrat fibreux en tissu non tissé

Le substrat fibreux, du type tissu non tissé, intervenant dans le cadre de l'invention est généralement choisi parmi ceux produits selon les techniques connues tels que, par exemple, ceux réalisés avec des fibres ou fils d'origine naturelle, artificielle ou synthétique, par les méthodes textiles, ou papetiers, dont les fibres, fils et couches fibreuses peuvent être liés par tous moyens appropriés tels que, par exemple, fibres fusibles incorporées devenant liantes par chauffage, colles adhésives, aiguilletage, ou autres.

Le substrat fibreux, du type tissu non tissé, intervenant dans le cadre de l'invention peut être formé au moyen de fibres ou fils constitutifs ayant un titre allant de 0,7 à 3,5 deniers, plus précisément allant de 0,7 à 3 deniers, et préférentiellement compris entre 0,7 et 2 deniers.

L'utilisation de fibres ayant un titre inférieur à 0,7 denier pour la formation d'un tissu non tissé pourrait conduire à l'apparition de difficultés techniques majeures qui rendraient difficile et coûteuse sa fabrication, alors que l'utilisation de fibres ou fils ayant un titre supérieur à 3,5 deniers pour la formation du tissu non tissé pourrait donner un touche insuffisamment textile qui se retrouverait dans la structure composite stratifiée te contenant.

Le substrat fibreux, du type tissu non tissé, mis en oeuvre dans le cadre de l'invention peut être réalisé au moyen de fils continus ou de fibres textiles, en particulier d'origine synthétique, tels que fils ou fibres de polyoléfines et, plus précisément, les fibres de polyéthylène, de polypropylène, fils ou fibres de polyesters, fils ou fibres de polyamide, fils ou fibres d'alcool polyvinylique.

Le substrat fibreux, du type tissu non tissé, mis en oeuvre dans le cadre de l'invention a un poids par unité de surface qui peut être compris entre 10 g/m² et 35 g/m², mais préférentiellement compris entre 13 g/m² et 18 g/m².

L'invention sera mieux comprise grâce aux figures illustratives.

La figure 1 est une couche schématique en coupe d'une structure composite stratifiée obtenue par laminage des couches constitutives en présence des fibrilles adhésives produites in situ.

La figure 2 est une coupe schématique du contact contrôlé entre le moyen de formation in situ des fibrilles adhésives et la couche en défilement linéaire qui est, dans le cas présent, un substrat fibreux.

La figure 3 est une coupe schématique de la position en contact contrôlé du moyen de formation in situ des fibrilles et de la couche en défilement, qui est un substrat fibreux avec création d'une indentation.

La figure 4 est une vue schématique représentative après laminage des fibrille adhésives produites in situ sur la couche en défilement.

La figure 5 est une photographie après laminage des fibrilles adhésives produites in situ sur la couche en défilement, qui est un substrat fibreux, à raison de 2,5 g/m²_{.}

La figure 6 est une photographie après laminage des fibrilles adhésives produites in situ sur la couche en défilement, qui est un substrat fibreux, à raison de 11,5 g/m².

La figure 7 est une photographie après laminage des fibrilles adhésives produites in situ sur la couche en défilement, qui est un substrat fibreux, à raison de 0,5 g/m².

La figure 8 est une photographie après laminage des fibrilles adhésives produites in situ sur la couche en défilement, qui est un substrat fibreux, à raison de 2 g/m².

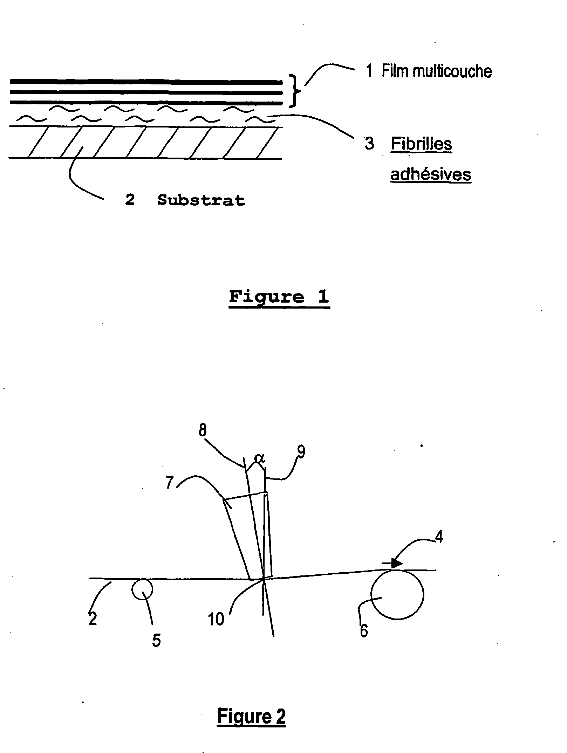

Selon la figure 1, la structure composite stratifiée, obtenue selon le procédé de l'invention par laminage des couches constitutives, comporte un film thermoplastique multicouches (1), microporeux, imperméable aux liquides mais perméable aux gaz et à la vapeur d'eau; un substrat fibreux du type tissu non tissé (2) formant la couche sur laquelle ont été produites in situ les fibrilles adhésives (3).

Selon la figure 2, qui est une vue schématique en coupe du contact physique contrôlé entre le moyen de formation des fibrilles adhésives in situ (7) et le substrat fibreux (2), ledit substrat (2) défile selon la flèche (4) entre les cylindres supports (5) et (6). La filière à fente (7), qui délivre la composition polymère permettant la formation des fibrilles adhésives in situ est en contact physique contrôlé avec le substrat fibreux (2). La position spatiale de ladite filière se définit par l'intermédiaire des deux plans (8) et (9), dont le plan (8), qui passe par la fente de la filière (7), est perpendiculaire à l'axe de symétrie du substrat fibreux (2) en défilement, et dont le plan (9) est perpendiculaire simultanément à l'axe de symétrie du substrat fibreux (2) et à la surface de défilement de cette même couche. Les deux plans (8) et (9) sont concourants en leur intersection (10) du substrat fibreux et forment entre eux un dièdre ayant un angle d'ouverture α compris entre 0° et 10°.

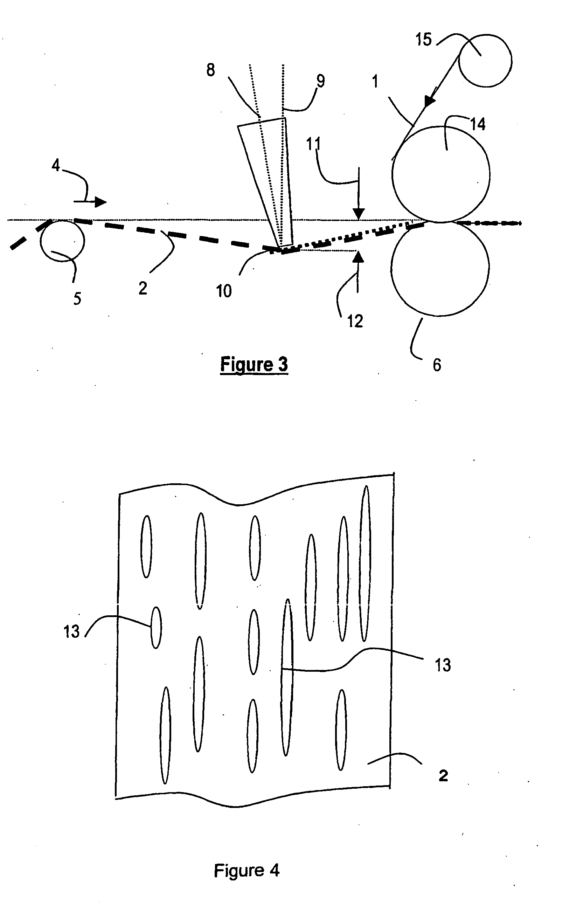

Selon la figure 3, qui est une vue schématique en coupe du contact physique contrôlé entre la filière à fente (7) et le substrat fibreux (2) avec création d'une indentation sur l'intersection (10) de deux plans précités (8) et (9), la filière, à fente (7) est en contact contrôlé en (10) par son abaissement et sa prise d'appui sur le substrat fibreux (2) en défilement entre les cylindres (5) et (6). L'importance de l'indentation est mesurée par l'écart existant entre les flèches (11) et (12). Immédiatement après la création in situ des fibrilles sur le substrat fibreux (2), la structure composite stratifiée selon l'invention est obtenue par l'assemblage au moyen du laminage entre les cylindres (6) et (14) du substrat fibreux (2) supportant les fibrilles adhésives et du film thermoplastique (15).

Selon la figure 4, qui est une vue schématique représentative des fibrilles adhésives produites in situ sur le substrat fibreux (2), les fibrilles adhésives (13) apparaissent distribuées de façon aléatoire sur le substrat fibreux et avoir des longueurs aléatoires.

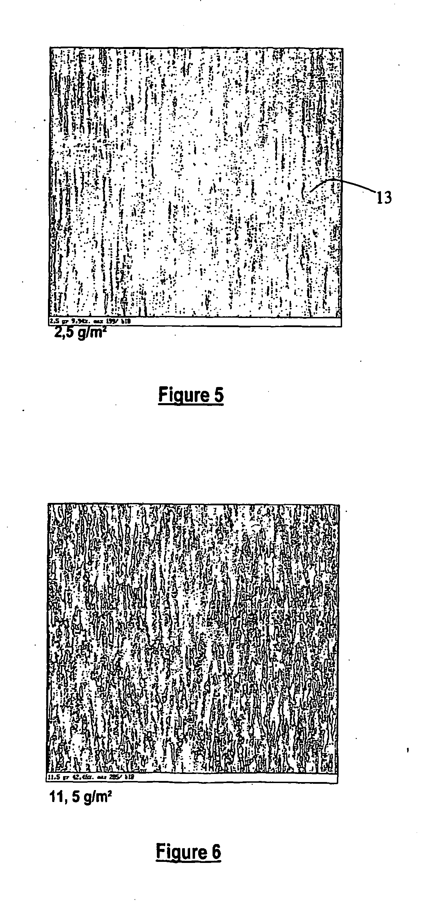

Selon la figure 5, qui est une photographie agrandie des fibrilles adhésives produites in situ sur le substrat fibreux (2) selon l'invention, la disposition des fibrilles adhésives et leur taille apparaissent absolument aléatoires, le grammage des fibrilles adhésives disposé sur le substrat fibreux étant de 2,5 g/m² et correspondant à une surface occupée d'environ 10% de la surface totale. La structure composite stratifiée correspondante est d'une excellente cohésion intercouche, est souple et d'un toucher très doux.

Selon la figure 6, qui est une photographie agrandie des fibrilles adhésives produites in situ sur le substrat fibreux (2), les fibrilles apparaissent comme des surfaces dont les dimensions (longueur et largeur) sont beaucoup plus importantes que celles visibles sur la figure 5, le grammage de liant adhésif déposé sur le substrat fibreux étant de 11,5 g/m² et représentant une surface occupée d'environ 30% de la surface totale. La structure composite stratifiée correspondante est d'une excellente cohésion intercouche, mais beaucoup moins souple et d'un toucher plus rêche que dans le cas de la figure 5.

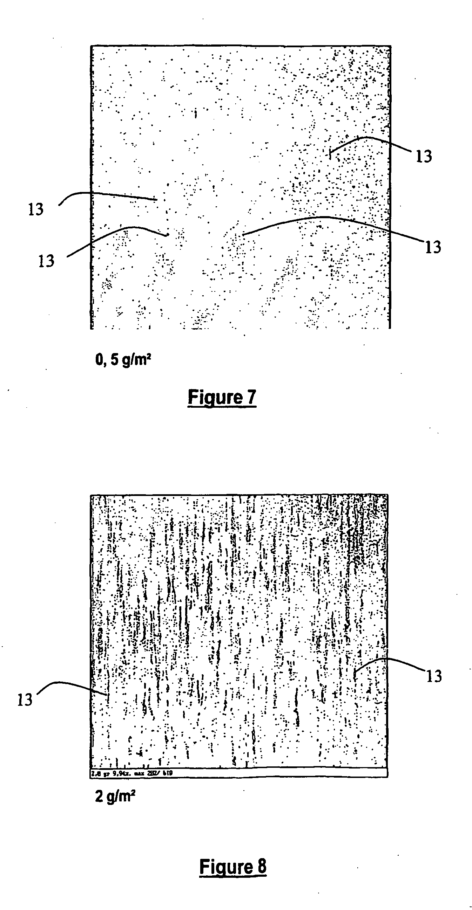

Selon la figure 7 qui est une photographie agrandie des fibrilles adhésives produites in situ sur le substrat fibreux (2), les fibrilles apparaissent comme quasiment des points ou encore de minuscules segments de longueur aléatoire, distribués de manière aléatoire sur la surface du substrat fibreux. Le grammage de liant adhésif déposé sur ledit substrat fibreux est de 0,5 g/m2 et représente une surface occupée d'environ 1,9% de la surface totale. La structure composite stratifiée correspondante est d'une bonne cohésion, est très souple et d'un toucher très doux.

### Exemple de réalisation (illustré par la figure 8)

On a réalisé une structure composite stratifiée selon l'invention se composant d'un film thermoplastique polyoléfinique microporeux multicouches, d'un substrat fibreux du type tissu non tissé et des fibrilles adhésives produites in situ intervenant comme agent liant entre les couches.

Le film thermoplastique mis en oeuvre est un film multicouches de structure du type "B-B-B-B-B", chaque couche "B" étant de même épaisseur et de même composition polyoléfinique.

Le film multicouches est obtenu par coextrusion d'un mélange de polymères fondus et de charge minérale dont la composition du mélange était :
- 44% de Dowlex 2035,
- 8% de copolymère bloc Adflex X102S,
- 48% d'une charge minérale : Filmlink 520.

Les différents matériaux cités sont décrits ci-dessous :
- le "Dowlex 2035" de DOW est un polyéthylène linéaire à basse densité à base d'octène ayant un indice de fluidité de 6,0 g/10 min (MFI norme ASTM D 1238 - 2,16 kg -190° C) et une densité de 0,919 (kg/m³) ;
- l'"Adflex X102S" de MONTELL est un polymère polyoléfinique thermoplastique avec un indice de fluidité de 8,0 g/10 min (MFI norme ASTM D 1238 - 2,16 kg - 230° C) et une densité de 0,890 (kg/m³) et un point de ramollissement de Vicat de 55° C ;
- "Filmlink 520" de ENGLISH CHINA CLAY (ECC) est une charge de carbonate de calcium (CaCO₃) broyé humide ayant un diamètre moyen D50 des particules de 2,0 microns. Elle possède une enveloppe hydrophobe de 1% et un Elrepho (brillance ISO) de 90.

Le film multicouches coextrudé a été étiré coaxialement selon un rapport d'étirage de 2,5/1. L'épaisseur du film multicouches étiré était de 15 µm. Son imperméabilité aux liquides a été contrôlé selon la norme EN20811-1992 et était de 1000 mm d'eau, et sa microporosité mesurée par la transmission de vapeur à 38° C et sous 90% d'humidité relative était de 2800 g/m²/24 h (selon la méthode Lyssy Apparatus Model 5000).

Le substrat fibreux mis en oeuvre était un tissu non tissé à base de polypropylène commercialisé par Fibertex, ayant un grammage de 15 g/m².

Le liant adhésif mis en oeuvre dans le cadre de l'invention était un hot melt commercialisé par National Starch and Chemical, sous l'appellation DISPOMELT 250, caractérisé par une viscosité Brookfield RVT (Module 27, vitesse 20 selon la méthode LC011) de 2800 mPas à 175° C. Ce hot melt était, selon l'invention, transformé in situ en fibrilles adhésives, de longueur aléatoire, distribuées de manière aléatoire sur la surface du tissu non tissé en défilement, à raison de 2,0 g/m² dudit tissu non tissé.

Les fibrilles adhésives formées in situ l'étaient à partir d'un applicateur de type Nordson, placé dans une chaîne de laminage juste en amont du moyen de laminage. La filière à fente avait une ouverture de fente de 0,3 mm et une largeur de 700 mm. La vitesse de défilement du tissu non tissé était de 317 m/min.

La filière à fente était en contact contrôlé avec le tissu non tissé :
- par l'intermédiaire de l'angle de dièdre α qui avait une valeur de 2° ;
- par la création d'une indentation de profondeur de 3 mm.

La température de la filière à fente était maintenue à 142° C et était alimentée en composition constitutive des fibrilles adhésives par l'intermédiaire d'une pompe dont le débit était asservi à la vitesse de défilement du tissu non tissé.

Le tissu non tissé était soumis à une tension de 1000 g lors de son défilement au contact contrôlé de la filière.

Le caractère aléatoire de la distribution des fibrilles adhésives produites in situ sur la surface du tissu non tissé a été confirmée par une observation de cette couche après l'obtention par laminage de la structure composite stratifiée selon l'invention et libération de ladite couche par délaminage.

| | **Film** | **Poids en gramme de fibres adhésive par unité de surface** | | |
|---|---|---|---|---|
| | | 1,0 g/m² | 1,5 g/m² | 2,0 g/m² |
| Transmission de vapeur d'eau en g/m² mesurée à 38°C et 90% d'humidité relative | 4500 | 4200 | 4200 | 4100 |
| Force de délaminage nécessaire pour séparer le film de la structure composite | | 0,55 | 0,9 | 1,45 |
| % de la surface de la couche fibreuse recouverte par les fibrilles adhésives | | 4,1 | 6,8 | 9,2 |
| Imperméabilité aux liquides mesurée en *mm pour une colonne hydrostatique* | Adéquate | Adéquate | Adéquate | Adéquate |
| Douceur au toucher | | Pas de perte | Pas de perte | Pas de perte |

Les structures composites stratifiées selon l'invention offrent simultanément toutes les caractéristiques ciblées dans le cadre de cette invention, en particulier une agréable douceur au toucher, une très grande souplesse et une bonne capacité à résister au délaminage.

## Revendications

1. Procédé continu de production d'une structure composite stratifiée laminée comprenant au moins deux couches dont l'une est formée d'un film thermoplastique imperméable aux liquides et l'autre est formée d'un substrat fibreux du type tissu non tissé, a) les couches étant rendues adhérentes entre elles par leur laminage en présence d'un moyen liant, **caractérisé en ce que** le moyen liant est formé par des fibrilles d'adhésion (3) produites in situ à partir d'au moins un polymère thermoplastique sur l'une des couches, immédiatement avant que s'effectue la lamination des au moins deux couches, par un moyen de formation (7) par extrusion en contact physique contrôlé avec la couche recevant les fibrilles.

2. Procédé selon la revendication 1, **caractérisé en ce que** les fibrilles d'adhésion produites in situ le sont préférentiellement sur le substrat fibreux (2).

3. Procédé selon l'une des revendications 1 ou 2, **caractérisé en ce que** les fibrilles adhésives (3), produites in situ, sont formées à partir de polymères et/ou copolymères thermoplastiques appartenant au groupe constitué par les polymères et/ou copolymères, amorphes ou non, de styrène-Isoprène-styrène, de styrène-butadiène-styrène, d'éthylène et propylène, d'éthylène et/ou de propylène avec au moins une alpha oléfine en C₄ à C₁₀, utilisés seuls ou en mélange.

4. Procédé selon l'une des revendications 1 ou 2, **caractérisé en ce que** les fibrilles adhésives produites in situ sont formées d'au moins 20% en poids, et préférentiellement de 30% à 100% en poids, de poly-α-oléfines amorphes appartenant au groupe constitué par les copolymères d'éthylène et propylène amorphes, par les copolymères d'éthylène et d'α-oléfines en C₄ à C₁₀ amorphes et les copolymères de propylène et d'α-oléfines en C₄ à C₁₀ amorphes, utilisés seuls ou en mélange.

5. Procédé selon l'une des revendications 1 ou 2, **caractérisé en ce que** les fibrilles adhésives produites in situ sont formées préférentiellement de 30% à 100% en poids, de poly-α-oléfines amorphes appartenant au groupe constitué par les copolymères d'éthylène et propylène amorphes, par les copolymères d'éthylène et d'α-oléflnes en C₄ à C₁₀ amorphes et les copolymères de propylène et d'α-oléfines en C₄ à C₁₀ amorphes, utilisés seuls ou en mélange.

6. Procédé selon l'une quelconque des revendications 3 à 5, **caractérisé en ce que** les fibrilles adhésives (3) produites in situ le sont à partir de polymères ou copolymères ayant souhaitablement une viscosité à l'état fondu comprise entre 2500 et 3000 à une température de 175° C (mesurée avec un viscosimètre Brookfield RVT).

7. Procédé selon l'une quelconque des revendications 1 à 6, **caractérisé en ce que** les fibrilles adhésives (3), produites in situ, ont une longueur distribuée de façon aléatoire d'au plus 20 mm et un rapport entre leur longueur et leur largeur après lamination comprise entre 1 et 100.

8. Procédé selon l'une quelconque des revendications 1 à 6, **caractérisé en ce que** les fibrilles adhésives (3), produites in situ, ont une longueur distribuée de façon aléatoire préférentiellement composes entre 0,1 et 10 mm, et un rapport entre leur longueur et leur largeur après lamination comprise entre 1 et 100.

9. Procédé selon l'une quelconque des revendications 1 à 8, **caractérisé en ce que** la moyen de formation utilisé pour la production in situ des fibrilles adhésives (3) immédiatement avant que s'effectue la lamination des diverses couches, est préférentiellement une filière à fente (7).

10. Procédé selon la revendication 9, **caractérisé en ce que** la formation in situ des fibrilles adhésives (3) est obtenue par la combinaison d'une sous-alimentation de la filière à fente (7), en composition polymère destinée à cette formation et de la vitesse linéaire de défilement de la couche sur laquelle sont produites lesdites fibrilles (3).

11. Procédé selon la revendication 10. **caractérisé en ce que** la vitesse linéaire de défilement de la couche recevant les fibrilles adhésives (3) produites in situ est comprise entre 50 m/min et 500 m/min.

12. Procédé selon la revendication 10, **caractérisé en ce que** la vitesse linéaire de défilement de la couche recevant les fibrilles adhésives (3) produites in situ est préférentiellement comprise entre 150 m/min et 300 m/min.

13. Procédé selon l'une quelconque des revendications 1 à 12, **caractérisé en ce que** la quantité de fibrilles adhésives (3) produites in situ sur la couche en défilement est d'au plus 6g/m².

14. Procédé selon l'une quelconque des revendications 1 à 12, **caractérisé en ce que** la quantité de fibrilles adhésives (3) produites in situ sur la couche en défilement est préférentiellement comprise entre 1,5 g/m² et 2,5 g/m².

15. Procédé selon l'une quelconque des revendications 1 à 14, **caractérisé en ce que** les fibrilles adhésives produites in situ sur la couche en défilement y sont distribuées d'une manière aléatoire.

16. Procédé selon l'une quelconque des revendications 1 à 15, **caractérisé en ce que** le moyen de formation des fibrilles (3), qui est en contact contrôlé avec la couche qui reçoit les fibrilles (3), occupe une position spatiale spécifique qui se définit par l'intermédiaire de deux plans concourants (8,9) dont l'intersection (10) se situe sur la couche recevant les fibrilles adhésives (3) perpendiculairement au sens de défilement de ladite couche, ces deux plans formant un dièdre d'angle a dont le premier plan, qui passe par le moyen de formation des fibrilles (3), est perpendiculaire à l'axe de symétrie de la couche recevant les fibrilles adhésives (3) et dont le deuxième plan est simultanément perpendiculaire à l'axe de symétrie de ladite couche et à la surface de défilement de cette même couche.

17. Procédé selon la revendication 16, **caractérisé en ce que** l'angle α, défini par les deux plans (8,9) concourants formant dièdre, prend une valeur de l'intervalle entre 10° et 0° en amont et par rapport au deuxième plan.

18. Procédé selon la revendication 16, **caractérisé en ce que** l'angle α, défini par les deux plans concourants (8,9) formant dièdre, prend une valeur de l'intervalle préférentiellement entre 2° et, 0° en amont et par rapport au deuxième plan.

19. Procédé selon l'une quelconque des revendications 16 à 18, **caractérisé en ce que** le contact contrôlé entre le moyen de formation in situ des fibrilles (3) et la couche en défilement recevant lesdites fibrilles (3) crée une indentation par abaissement et prise d'appui du moyen de formation (7) des fibrilles (3) sur la couche en défilement.

20. Procédé selon la revendication 19, **caractérisé en ce que** la profondeur de l'indentation dans la couche recevant les fibrilles adhésives (3), produites par abaissement et la prise d'appui du moyen de formation (7) in situ des fibrilles (3) sur ladite couche varie entre 0 et 5 mm.

21. Procédé selon la revendication 19, **caractérisé en ce que** la profondeur de l'indentation dans la couche recevant les fibrilles adhésives (3) produites par abaissement et la prise d'appui du moyen de formation (7) in situ des fibrilles (3) sur ladite couche varie préférentiellement entre 2 et 3 mm.

22. Procédé selon l'une quelconque des revendications 16 à 21, **caractérisé en ce que** la couche en défilement recevant les fibrilles adhésives (3) produites in situ est soumise à une tension Contrôlée comprise dans l'intervalle de 500 g à 2000 g.

23. Procédé selon l'une quelconque des revendications 16 à 21, **caractérisé en ce que** la couche en défilement recevant les fibrilles adhésives (3) produites in situ est soumise à une tension contrôlée préférentiellement comprise dans l'intervalle de 800 g à 1500 g.

24. Procédé selon l'une quelconque des revendications 1 à 23, **caractérisé en ce que** la température sous laquelle sont produites in situ les fibrilles adhésives (3) est comprise entre 120°C et 185°C.

25. Procédé selon l'une quelconque des revendications 1 à 23, **caractérisé en ce que** la température sous laquelle sont produites in situ les fibrilles adhésives (3) est comprise préférentiellement entre 140°C et 165°C.

26. Procédé selon l'une quelconque des revendications 1 à 25, **caractérisé en ce que** la surface de la couche recouverte par les fibrilles adhésives (3) produites in situ représente au plus 20% de la surface totale de ladite couche.

27. Procédé selon l'une quelconque des revendications 1 à 25, **caractérisé en ce que** la surface de la couche recouverte par les fibrilles adhésives (3) produites in situ représente préférentiellement de 5% à 10% de la surface totale de ladite couche.

28. Procédé selon l'une quelconque des revendications 1 à 27, **caractérisé en ce que** le film thermoplastique mis en oeuvre est formé à partir de polymères et/ou copolymères choisis dans le groupe formé par les polyoléfines, les polyesters, les polyuréthanes, les alcools polyvinyliques.

29. Procédé selon la revendication 28, **caractérisé en ce que**, quand le film thermoplastique est polyoléfinique, il est formé à partir de polymères et/ou copolymères issus du groupe constitué par les polyéthylènes de haute densité, les polyéthylènes de basse densité, les copolymères d'éthylène et d'α-oléflnes en C₄ à C₁₀, les polypropylènes, les copolymères de propylène et d'α-oléfines en C₄ à C₁₀, les copolymères d'éthylène-propylène, seuls ou en mélange.

30. Procédé selon l'une quelconque des revendications 1 à 29, **caractérisé en ce que** le film thermoplastique a une épaisseur comprise entre 10µm et 30µm.

31. Procédé selon l'une quelconque des revendications 1 à 29, **caractérisé en ce que** le film thermoplastique a une épaisseur préférentiellement comprise entre 13µm et 20µm.

32. Procédé selon l'une quelconque des revendications 1 à 31, **caractérisé en ce que** le film thermoplastique imperméable aux liquides est perméable ou non aux gaz et à la vapeur d'eau.

33. Procédé selon la revendication 32, **caractérisé en ce que**, quand le film thermoplastique. est perméable aux gaz et à la vapeur d'eau, la perméabilité du film thermoplastique est préférentiellement obtenue par le mise en oeuvre d'une charge minérale dans la composition du film et l'étirage dudit film chargé.

34. Procédé selon la revendication 33, **caractérisé en ce que** la perméabilité du film thermoplastique est souhaitablement comprise entre 500 g/m²/24 h et 5000 g/m²/24 h, cette perméabilité étant mesurée sous les conditions de température de 38° C et d'humidité relative de 90% (selon ia méthode "Lyssy apparatus model 5000").

35. Procédé selon la revendication 33, **caractérisé en ce que** la perméabilité du film thermoplastique est préférentiellement comprise entre 2000 g/m²/24 h et 5000 g/m²/24h, cette perméabilité étant mesurée sous les conditions de température de 38° C et d'humidité relative de 90% (selon la méthode "Lyssy apparatus model 5000°).

36. Procédé selon l'une quelconque des revendications 1 à 35, **caractérisé en ce que** le film thermoplastique est monocouche ou multicouches.

37. Procédé selon l'une quelconque des revendications 1 à 36, **caractérisé en ce que** le substrat fibreux (2) du type tissu non tissé, est choisi parmi ceux produits selon les techniques connues avec des fibres ou fils d'origine naturelle, artificielle ou synthétique, par les méthodes textiles, ou papetiers.

38. Procédé selon l'une quelconque des revendications 1 à 37. **caractérisé en ce que** le substrat fibreux (2), du type tissu non tissé, est formé au moyen de fibres ou fils constitutifs ayant un titre allant de 0,7 à 3,5 deniers.

39. Procédé selon l'une quelconque des revendications 1 à 38, **caractérisé en ce que** le substrat fibreux (2), du type tissu non tissé, est formé préférentiellement de fils ou fibres de polyéthylène, de polypropylène, fils ou fibres de polyesters, fils ou fibres de polyamide, fils ou fibres d'alcool polyvinylique.

40. Procédé selon l'une quelconque des revendications 1 à 39, **caractérisé en ce que** le substrat fibreux (2), du type tissu non tissé, a un poids par unité de surface qui est compris entre 10 g/m² et 35 g/m².

41. Procédé selon l'une quelconque des revendications 1 à 39, **caractérisé en ce que** le substrat fibreux (2), du type tissu non tissé, a un poids par unité de surface qui est préférentiellement compris entre 13 g/m² et 18 g/m².

42. Structure composite stratifiée comprenant au moins deux couches dont l'une est formée d'un film thermoplastique imperméable aux liquides, et l'autre est formée d'un substrat fibreux (2), du type tissu non tissé, les couches étant rendues adhérentes entre elles par laminage en présence d'un liant, **caractérisée en ce que** le liant est constitué par des fibrilles adhésives (3) produites in situ sur l'une des couches, selon l'une au moins des revendications 1 à 27, immédiatement avant la lamination.

43. Structure composite selon la revendication 42, dans laquelle le film thermoplastique répond à l'une au moins des revendications 28 à 36.

44. Structure composite selon la revendication 42, dans laquelle le substrat fibreux (2) répond à l'une au moins des revendications 37 à 41.

45. Application de la structure composite stratifié selon la revendication 42, en particulier dans le domaine des articles d'hygiène jetables, tels que couches, hygiène féminine, articles dans le domaine médical.

## Claims

1. A continuous process for manufacturing a laminated composite sheet structure (1) comprising at least two layers, one of which being formed of a liquid impermeable thermoplastics film and the other being formed of a fibrous web of the non woven type (2), the layers being made adherent to each other by lamination in the presence of a binding means,
**characterized in that** the binding means is made of adhesion fibrils (3) formed in-situ from at least one thermoplastic polymer on one of the at least two layers, immediately before the lamination of the at least two layers takes place, by a formation means (7) by extrusion in controlled physical contact with the fibrils receiving layers.

2. Process according to claim 1 **characterized in that** the adhesion fibrils (3) formed in situ are formed preferably on the fibrous web (2).

3. Process according to claim 1 or 2 **characterized in that** the adhesive fibrils (3) formed in situ are formed from thermoplastic polymers and / or copolymers belonging to the group consisting of amorphous or non amorphous polymers and / or copolymers of styrene-isoprene-styrene, styrenebutadiene-styrene, ethylene and propylene, ethylene and / or propylene with at least one C4 to C10 alpha olefin, used alone or in blends.

4. Process according to claim 1 or 2 **characterized in that** the adhesive fibrils (3) formed in situ are formed from at least 20 % by weight and preferably from 30 % to 100 % by weight of amorphous poly-α-olefin belonging to the group consisting of ethylene and propylene amorphous copolymers, by amorphous copolymers of ethylene and at least one C₄ to C₁₀ α-olefins and by amorphous copolymers of propylene and at least one C₄ to C₁₀ α-olefins, used alone or in blend.

5. Process according to claim 1 or 2 **characterized in that** the adhesive fibrils (3) formed in situ are formed from preferably from 30 % to 100 % by weight of amorphous poly-α-olefin belonging to the group consisting of ethylene and propylene amorphous copolymers, by amorphous copolymers of ethylene and at least one C₄ to C₁₀ α-olefins and by amorphous copolymers of propylene and at least one C₄ to C₁₀ α-olefins, used alone or in blend.

6. Process according to any one of claims 3 to 5 **characterized in that** the adhesive fibrils (3) formed in situ are formed from polymers or copolymers having a desired melt viscosity of from 2500 to 3000 mPa.s, at 175 °C, when measured according to Brookfield RVT viscosimeter.

7. Process according to any one of claims 1 to 6 **characterized in that** the adhesive fibrils (3) formed in situ have a randomly distributed length of at most 20 mm and a ratio between their length and their width after lamination comprised between 1 and 100.

8. Process according to any one of claims 1 to 6 **characterized in that** the adhesive fibrils (3) formed in situ have a randomly distributed length preferably comprised between 0,1 mm and 10 mm and a ratio between their length and their width after lamination comprised between 1 and 100.

9. Process according to any one of claims 1 to 8 **characterized in that** the formation means used to produce in situ the adhesive fibrils (3) immediately before the lamination of the various layers takes place, is preferably a slot die (7).

10. Process according to claim 9 **characterized in that** the in situ formation of the adhesive fibrils (3) is achieved by the combination of the under-feeding the slot die (7) with polymer combination and of the linear moving speed of the layer onto which said fibrils are produced.

11. Process according to claim 10 **characterized in that** the linear moving speed of the layer receiving the in situ formed adhesive fibrils (3) is comprised between 50 m/min and 500 m/min.

12. Process according to claim 10 **characterized in that** the linear moving speed of the layer receiving the in situ formed adhesive fibrils (3) is comprised preferably between 150 m/min and 300 m/min.

13. Process according to any one of claims 1 to 12
**characterized in that** the quantity of in situ formed adhesive fibrils (3) on the moving layer is at most equal to 6 g/m².

14. Process according to any one of claims 1 to 12
**characterized in that** the quantity of in situ formed adhesive fibrils (3) on the moving layer is preferably comprised between 1,5 g/m² and 2,5 g/m².

15. Process according to any one of claims 1 to 14
**characterized in that** the in situ formed adhesive fibrils are randomly distributed on the moving layer surface.

16. Process according to any one of claims 1 to 15
**characterized in that** the fibrils (3) formation means, which is controlled contact with the fibrils (3) receiving layer, is in a specific spatial position, defined thanks to two converging planes (8, 9), the intersection (10) of which is situated on the adhesive fibrils (3) receiving layer, perpendicular to the moving direction of said layer, these two planes forming a dihedral of angle α, the first plane of which, extending through the fibrils (3) formation means, is perpendicular to the symmetry axis of the adhesive fibrils (3) receiving layer, and the second plane of which is simultaneously perpendicular to said layer symmetry axis and to the moving surface of the same layer.

17. Process according to claim 16 **characterized in that** the angle a defined by the two converging planes (8, 9) forming the dihedral is comprised between 10° and 0° and is situated upstream relative to the second plane.

18. Process according to claim 16 **characterized in that** the angle α defined by the two converging planes (8, 9) forming the dihedral is comprised preferably between 2° and 0° and is situated upstream relative to the second plane.

19. Process according to any one of claims 16 to 18 **characterized in that** the controlled contact between the in situ fibrils (3) formation means (7) and the moving layer receiving said fibrils (3) creates an indentation by lowering and pressing of the fibrils (3) formation means onto the moving layer.

20. Process according to claim 19 **characterized in that** the depth of indentation in the moving web receiving the adhesive fibrils (3), produced by lowering and pressing of the in situ formed fibrils (3) formation means (7) onto said moving web, varies between 0 and 5 mm.

21. Process according to claim 19 **characterized in that** the depth of indentation in the moving web receiving the adhesive fibrils (3), produced by lowering and pressing of the in situ formed fibrils (3) formation means (7) onto said moving web, varies preferably between 2 and 3 mm.

22. Process according to any one of claims 16 to 21
**characterized in that** the moving layer receiving the adhesive fibrils (3) produced in situ is subjected to a controlled tension comprised between 500 g and 2000 g.

23. Process according to any one of claims 16 to 21
**characterized in that** the moving layer receiving the adhesive fibrils (3) produced in situ is subjected to a controlled tension comprised preferably between 800 and 1500 g.

24. Process according to any one of claims 1 to 23
**characterized in that** the adhesive fibrils (3) are formed in situ at a temperature comprised between 120°C and 185 °C.

25. Process according to any one of claims 1 to 23
**characterized in that** the adhesive fibrils (3) are formed in situ at a temperature comprised between preferably between 140°C and 165°C.

26. Process according to any one of claims 1 to 25
**characterized in that** the surface of the layer covered by the in situ formed adhesive fibrils (3) is at most 20 % of the total surface of said layer.

27. Process according to any one of claims 1 to 25
**characterized in that** the surface of the layer covered by the in situ formed adhesive fibrils (3) is preferably from 5% to 10 % of the total surface of said layer.

28. Process according to any one of claims 1 to 27
**characterized in that** the thermoplastic film used is formed from polymers and/or copolymers selected the group consisting of polyolefins, polyesters, polyurethanes, polyvinyl alcohols.

29. Process according to claim 28 **characterized in that**, when the thermoplastic film is of a polyolefinic composition, it is formed from polymers and / or copolymers selected from the group consisting of high density polyethylene, low density polyethylene, copolymers of ethylene and C₄ to C₁₀ α-olefin, polypropylene, copolymers of propylene and C₄ to C₁₀ α-olefin, copolymers of ethylene propylene, alone or in blends.

30. Process according to any one of claims 1 to 29 **characterized in that** the thermoplastic film has a thickness of 10 µm to 30 µm.

31. Process according to any one of claims 1 to 29 **characterized in that** the thermoplastic film has a thickness preferably between 13 µm and 20 µm.

32. Process according to any one of claims 1 to 31 **characterized in that** the liquid impermeable thermoplastic film is permeable or not to gases and water vapor.

33. Process according to claim 32 **characterized in that**, when the thermoplastic film is permeable to gases and water vapor, the permeability of the thermoplastic film is preferably obtained by adding a mineral filler in the film composition and subjecting said filler loaded film to stretching.

34. Process according to claim 33 **characterized in that** the
permeability of the thermoplastic film is desirably comprised between 500 g/m²/24h and 5000 g/m²/24h, when measured under temperature conditions of 38 °C, at 90 % relative humidity according to the method using the Lissy apparatus model 5000.

35. Process according to claim 33 **characterized in that** the
permeability of the thermoplastic film is preferably comprised between 2000 g/m²/24h and 5000 g/m²/24h, when measured under temperature conditions of 38 °C, at 90 % relative humidity according to the method using the Lissy apparatus model 5000.

36. Process according to any one of claims 1 to 35
**characterized in that** the thermoplastic film is a mono or a multilayer film.

37. Process according to any one of claims 1 to 26
**characterized in that** the fibrous substrate (2) of the non woven type, is selected from those produced by known means using natural or synthetic fibers or filaments, processed according to textile or paper making techniques.

38. Process according to one at least of claims 1 to 37
**characterized in that** the fibrous substrate (2) of the non woven type, is formed by means of fibers or filaments of 0,7 to 3,5 deniers.

39. Process according to one at least of claims 1 to 38 **characterized in that** the fibrous substrate (2) of the non woven type, is formed preferably of fibers or filaments of polyethylene, or polypropylene, or polyester, or polyamide, or polyvinyl alcohol.

40. Process according to one at least of claims 1 to 39 **characterized in that** the fibrous substrate (2) of the non woven type, has a weight per unit surface comprised between 10 g/m² and 35 g/m².

41. Process according to one at least of claims 1 to 39 **characterized in that** the fibrous substrate (2) of the non woven type, has a weight per unit surface comprised preferably between 13 g/m² and 18 g/m².

42. Composite laminated sheet structure comprising at least two layers one of which being formed of a liquid impermeable thermoplastic film, the other being formed of a fibrous web (2) of the non woven type, the layers being made adherent to each other by means of lamination in the presence of a binder, **characterized in that** the binder is constituted by in-situ formed adhesive fibrils (3) created on one of the layers, according to at least one of the claims 1 to 27, immediately before lamination.

43. Composite laminated sheet structure according to claim 42, in which the thermoplastic film is in accordance with at least one of the claims 28 to 36.

44. Composite laminated structure according to claim 42, in which the fibrous web (2) is in accordance with at least one of the claims 37 to 41.

45. Application of the composite laminated structure according to claim 42, in particular in the domain of disposable hygiene articles, such as diapers, feminine hygiene articles, medical articles.

## Patentansprüche

1. Verfahren zur kontinuierlichen Herstellung einer laminierten Schichtverbundstruktur (1) bestehend aus mindestens zwei Schichten, wovon eine Schicht von einem flüssigkeitsundurchlässigen thermoplastischen Film gebildet ist und die andere Schicht aus einem vliesstoffartigen Fasersubstrat (2) besteht, wobei die Schichten durch Laminieren in Anwesenheit eines Bindemittels haftend miteinander verbunden werden, **dadurch gekennzeichnet, dass** das Bindemittel aus Haftfibrillen (3) besteht, die in situ aus mindestens einem thermoplastischen Polymer auf einer der Schichten unmittelbar vor dem Laminieren der mindestens zwei Schichten unter Verwendung eines Mittels (7) zur Herstellung durch Strangpressen erzeugt werden, das mit der die Fibrillen empfangenden Schicht in kontrolliertem physischen Kontakt steht.

2. Verfahren nach Anspruch 1, **dadurch gekennzeichnet, dass** die in situ erzeugten Haftfibrillen (3) dabei vorzugsweise auf dem Fasersubstrat (2) erzeugt werden.

3. Verfahren nach einem der Ansprüche 1 oder 2, **dadurch gekennzeichnet, dass** die in situ erzeugten Haftfibrillen (3) aus thermoplastischen Polymeren bzw. Copolymeren hergestellt werden, die der Gruppe der amorphen oder nicht amorphen Styrol-Isopren-Styrol-, Styrol-Butadien-Styrol-, Ethylen- und Propylen-, Ethylen und/oder Propylen-Polymere bzw. -Copolymere mit mindestens einem Alphaolefin im Bereich von C₄ bis C₁₀ angehören und einzeln oder in Mischung eingesetzt werden.

4. Verfahren nach einem der Ansprüche 1 oder 2, **dadurch gekennzeichnet, dass** die in situ erzeugten Haftfibrillen (3) aus mindestens 20 Gew.-%, bevorzugt 30 bis 100 Gew.-% amorpher Polyalphaolefine bestehen, die der Gruppe der amorphen Ethylen- und Propylen-Copolymere, der amorphen Ethylen- und C₄- bis C₁₀-Alphaolefin-Copolymere und der amorphen Propylen- und C₄- bis C₁₀-Alphaolefin-Copolymere angehören und einzeln oder in Mischung eingesetzt werden.

5. Verfahren nach einem der Ansprüche 1 oder 2, **dadurch gekennzeichnet, dass** die in situ erzeugten Haftfibrillen (3) bevorzugt aus 30 bis 100 Gew.-% amorpher Polyalphaolefine bestehen, die der Gruppe der amorphen Ethylen- und Propylen-Copolymere, der amorphen Ethylen- und C₄- bis C₁₀-Alphaolefin-Copolymere und der amorphen Propylen- und C₄- bis C₁₀-Alphaolefin-Copolymere angehören und einzeln oder in Mischung eingesetzt werden.

6. Verfahren nach irgendeinem der Ansprüche 3 bis 5, **dadurch gekennzeichnet, dass** die in situ erzeugten Haftfibrillen (3) aus Polymeren bzw. Copolymeren hergestellt werden, die wünschenswerter Weise eine Viskosität im schmelzflüssigen Zustand zwischen 2500 und 3000 mPa.s bei einer Temperatur von 175 °C haben (gemessen mit einem Brookfield-Viskosimeter RVT).

7. Verfahren nach irgendeinem der Ansprüche 1 bis 6, **dadurch gekennzeichnet, dass** die in situ erzeugten Haftfibrillen (3) eine aleatorisch verteilte Länge von höchstens 20 mm und ein Verhältnis von Länge zu Breite nach erfolgtem Laminieren zwischen 1 und 100 haben.

8. Verfahren nach irgendeinem der Ansprüche 1 bis 6, **dadurch gekennzeichnet, dass** die in situ erzeugten Haftfibrillen (3) eine aleatorisch verteilte Länge von vorzugsweise 0,1 bis 10 mm und ein Verhältnis von Länge zu Breite nach erfolgtem Laminieren zwischen 1 und 100 haben.

9. Verfahren nach irgendeinem der Ansprüche 1 bis 8, **dadurch gekennzeichnet, dass** das für die in situ-Erzeugung der Haftfibrillen (3) unmittelbar vor dem Laminieren der einzelnen Schichten eingesetzte Herstellungsmittel bevorzugt eine Schlitzdüse (7) ist.

10. Verfahren nach Anspruch 9, **dadurch gekennzeichnet, dass** die in situ-Herstellung der Haftfibrillen (3) **dadurch** erzielt wird, dass eine Unterversorgung der Schlitzdüse (7) mit Polymermischung für diese Herstellung mit der linearen Durchlaufgeschwindigkeit der Schicht, auf der die Fibrillen (3) erzeugt werden, kombiniert wird.

11. Verfahren nach Anspruch 10, **dadurch gekennzeichnet, dass** die lineare Durchlaufgeschwindigkeit der Schicht, die die in situ erzeugten Haftfibrillen (3) empfängt, zwischen 50 m/min und 500 m/min liegt.

12. Verfahren nach Anspruch 10, **dadurch gekennzeichnet, dass** die lineare Durchlaufgeschwindigkeit der Schicht, die die in situ erzeugten Haftfibrillen (3) empfängt, bevorzugt zwischen 150 m/min und 300 m/min liegt.

13. Verfahren nach irgendeinem der Ansprüche 1 bis 12, **dadurch gekennzeichnet, dass** die Menge der in situ erzeugten Haftfibrillen (3) auf der durchlaufenden Schicht höchstens 6g/m² beträgt.

14. Verfahren nach irgendeinem der Ansprüche 1 bis 12, **dadurch gekennzeichnet, dass** die Menge der in situ erzeugten Haftfibrillen (3) auf der durchlaufenden Schicht bevorzugt zwischen 1,5 g/m² und 2,5 g/m² liegt.

15. Verfahren nach irgendeinem der Ansprüche 1 bis 14, **dadurch gekennzeichnet, dass** die in situ erzeugten Haftfibrillen (3) auf der durchlaufenden Schicht dort aleatorisch verteilt werden.

16. Verfahren nach irgendeinem der Ansprüche 1 bis 15, **dadurch gekennzeichnet, dass** das Mittel zur Herstellung der Fibrillen (3), das in kontrolliertem Kontakt mit der die Fibrillen (3) empfangenden Schicht steht, eine spezifische räumliche Position einnimmt, die durch zwei zusammenlaufende Ebenen (8, 9) definiert ist, deren Schnittstelle (10) auf der die Fibrillen (3) empfangenden Schicht quer zur Durchlaufrichtung dieser Schicht liegt, wobei diese beiden Ebenen ein Dieder mit dem Winkel α bilden, dessen erste, durch das Mittel zur Herstellung der Fibrillen (3) gehende Ebene quer zur Symmetrieachse der die Haftfibrillen (3) empfangenden Schicht verläuft und dessen zweite Ebene gleichzeitig quer zur Symmetrieachse dieser Schicht und zu deren Durchlauffläche verläuft.

17. Verfahren nach Anspruch 16, **dadurch gekennzeichnet, dass** der Winkel α, der durch die beiden zusammenlaufenden, ein Dieder bildenden Ebenen (8, 9) definiert ist, oben und in Bezug auf die zweite Ebene einen Wert im Bereich zwischen 10° und 0° annimmt.

18. Verfahren nach Anspruch 16, **dadurch gekennzeichnet, dass** der Winkel α, der durch die beiden zusammenlaufenden, ein Dieder bildenden Ebenen (8, 9) definiert ist, oben und in Bezug auf die zweite Ebene bevorzugt einen Wert im Bereich zwischen 2° und 0° annimmt.

19. Verfahren nach irgendeinem der Ansprüche 16 bis 18, **dadurch gekennzeichnet, dass** durch den kontrollierten Kontakt zwischen dem Mittel (7) zur in situ-Herstellung der Fibrillen (3) und der durchlaufenden Schicht, die die Fibrillen (3) empfängt, eine Ausbuchtung infolge Absenken und Abstützen des Mittels (7) zur Herstellung der Fibrillen (3) auf der durchlaufenden Schicht entsteht.

20. Verfahren nach Anspruch 19, **dadurch gekennzeichnet, dass** die Tiefe der Ausbuchtung in der die Haftfibrillen (3) empfangenden Schicht infolge des Absenkens und des Abstützens des Mittels (7) zur in situ-Herstellung der Fibrillen (3) auf dieser Schicht zwischen 0 und 5 mm schwankt.

21. Verfahren nach Anspruch 19, **dadurch gekennzeichnet, dass** die Tiefe der Ausbuchtung in der die Haftfibrillen (3) empfangenden Schicht infolge des Absenkens und des Abstützens des Mittels (7) zur in situ-Herstellung der Fibrillen (3) auf dieser Schicht bevorzugt zwischen 2 und 3 mm schwankt.

22. Verfahren nach irgendeinem der Ansprüche 16 bis 21, **dadurch gekennzeichnet, dass** die durchlaufende Schicht, die die in situ erzeugten Haftfibrillen (3) empfängt, einer kontrollierten Spannung im Bereich von 500 g bis 2000 g unterworfen wird.

23. Verfahren nach irgendeinem der Ansprüche 16 bis 21, **dadurch gekennzeichnet, dass** die durchlaufende Schicht, die die in situ erzeugten Haftfibrillen (3) empfängt, einer kontrollierten Spannung bevorzugt im Bereich von 800 g bis 1500 g unterworfen wird.

24. Verfahren nach irgendeinem der Ansprüche 1 bis 23, **dadurch gekennzeichnet, dass** die Temperatur, bei der die Haftfibrillen (3) in situ erzeugt werden, zwischen 120 °C und 185 °C liegt.

25. Verfahren nach irgendeinem der Ansprüche 1 bis 23, **dadurch gekennzeichnet, dass** die Temperatur, bei der die Haftfibrillen (3) in situ erzeugt werden, bevorzugt zwischen 140 °C und 165 °C liegt.

26. Verfahren nach irgendeinem der Ansprüche 1 bis 25, **dadurch gekennzeichnet, dass** die Fläche der von den in situ erzeugten Haftfibrillen (3) bedeckten Schicht höchstens 20 % der Gesamtfläche dieser Schicht darstellt.

27. Verfahren nach irgendeinem der Ansprüche 1 bis 25, **dadurch gekennzeichnet, dass** die Fläche der von den in situ erzeugten Haftfibrillen (3) bedeckten Schicht bevorzugt 5 bis 10 % der Gesamtfläche dieser Schicht darstellt.

28. Verfahren nach irgendeinem der Ansprüche 1 bis 27, **dadurch gekennzeichnet, dass** der eingesetzte thermoplastische Film aus Polymeren bzw. Copolymeren, gewählt aus der Gruppe der Polyolefine, Polyester, Polyurethane, Polyvinylalkohole, hergestellt wird.

29. Verfahren nach Anspruch 28, **dadurch gekennzeichnet, dass** der thermoplastische Film, wenn er polyolefinisch ist, aus Polymeren bzw. Copolymeren aus der Gruppe der Polyethylene hoher Dichte, Polyethylene niedriger Dichte, Ethylen- und C₄- bis C₁₀-Alphaolefin-Copolymere, Polypropylene, Propylen- und C₄- bis C₁₀-Alphaolefin-Copolymere, Ethylen-Propylen-Copolymere hergestellt wird, die einzeln oder in Mischung eingesetzt werden.

30. Verfahren nach irgendeinem der Ansprüche 1 bis 29, **dadurch gekennzeichnet, dass** der thermoplastische Film eine Dicke zwischen 10 µm und 30 µm hat.

31. Verfahren nach irgendeinem der Ansprüche 1 bis 29, **dadurch gekennzeichnet, dass** der thermoplastische Film bevorzugt eine Dicke zwischen 13 µm und 20 µm hat.

32. Verfahren nach irgendeinem der Ansprüche 1 bis 31, **dadurch gekennzeichnet, dass** der flüssigkeitsundurchlässige thermoplastische Film gas- und wasserdampfdurchlässig ist oder nicht.

33. Verfahren nach Anspruch 32, **dadurch gekennzeichnet, dass** wenn der thermoplastische Film gas- und wasserdampfdurchlässig ist, die Durchlässigkeit des thermoplastischen Films vorzugsweise durch Einbringen eines mineralischen Füllstoffs in die Zusammensetzung des Films und Ziehen des angereicherten Films erzielt wird.

34. Verfahren nach Anspruch 33, **dadurch gekennzeichnet, dass** die Durchlässigkeit des thermoplastischen Films wünschenswerter Weise zwischen 500 g/m²/24 h und 5000 g/m²/24h liegt, wobei diese Durchlässigkeit unter Temperaturbedingungen von 38°C und relativen Feuchtigkeitsbedingungen von 90 % gemessen wird (nach der Methode "Lyssy apparatus model 5000").

35. Verfahren nach Anspruch 33, **dadurch gekennzeichnet, dass** die Durchlässigkeit des thermoplastischen Films bevorzugt zwischen 2000 g/m²/24 h und 5000 g/m²/24h liegt, wobei diese Durchlässigkeit unter Temperaturbedingungen von 38°C und relativen Feuchtigkeitsbedingungen von 90 % gemessen wird (nach der Methode "Lyssy apparatus model 5000").

36. Verfahren nach irgendeinem der Ansprüche 1 bis 35, **dadurch gekennzeichnet, dass** der thermoplastische Film ein- oder mehrschichtig ist.

37. Verfahren nach irgendeinem der Ansprüche 1 bis 36, **dadurch gekennzeichnet, dass** das vliesstoffartige Fasersubstrat (2) unter denjenigen gewählt wird, die nach den bekannten Techniken mit Fasern oder Fäden natürlicher, künstlicher oder synthetischer Herkunft, durch textiltechnische oder papiertechnische Methoden hergestellt werden.

38. Verfahren nach irgendeinem der Ansprüche 1 bis 37, **dadurch gekennzeichnet, dass** das vliesstoffartige Fasersubstrat (2) mittels konstitutiver Fasern oder Fäden mit einer Feinheit von 0,7 bis 3,5 Denier gebildet wird.

39. Verfahren nach irgendeinem der Ansprüche 1 bis 38, **dadurch gekennzeichnet, dass** das vliesstoffartige Fasersubstrat (2) bevorzugt aus Polyethylen-, Polypropylenfäden oder -fasern, Polyesterfäden oder -fasern, Polyamidfäden oder -fasern, Polyvinylalkoholfäden oder - fasern besteht.

40. Verfahren nach irgendeinem der Ansprüche 1 bis 39, **dadurch gekennzeichnet, dass** das vliesstoffartige Fasersubstrat (2) ein Gewicht pro Flächeneinheit hat, das zwischen 10 g/m² und 35 g/m² liegt.

41. Verfahren nach irgendeinem der Ansprüche 1 bis 39, **dadurch gekennzeichnet, dass** das vliesstoffartige Fasersubstrat (2) ein Gewicht pro Flächeneinheit hat, das bevorzugt zwischen 13 g/m² und 18 g/m² liegt.

42. Schichtverbundstruktur bestehend aus mindestens zwei Schichten, wovon eine Schicht von einem flüssigkeitsundurchlässigen thermoplastischen Film gebildet ist und die andere Schicht aus einem vliesstoffartigen Fasersubstrat (2) besteht, wobei die Schichten durch Laminieren in Anwesenheit eines Bindemittels haftend miteinander verbunden werden, **dadurch gekennzeichnet, dass** das Bindemittel aus Haftfibrillen (3) besteht, die nach mindestens einem der Ansprüche 1 bis 27 in situ auf einer der Schichten unmittelbar vor dem Laminieren erzeugt werden.

43. Verbundstruktur nach Anspruch 42, wobei der thermoplastische Film mindestens einem der Ansprüche 28 bis 36 genügt.

44. Verbundstruktur nach Anspruch 42, wobei das Fasersubstrat (2) mindestens einem der Ansprüche 37 bis 41 genügt.

45. Anwendung der Schichtverbundstruktur nach Anspruch 42, insbesondere im Bereich der Einweg-Hygieneartikel, wie Windeln, Frauenhygiene, Artikel für den medizinischen Bereich.
